# EUROPEAN PATENT SPECIFICATION

(11) **EP 2 058 318 B1**
(45) Date of publication and mention of the grant of the patent: **16.05.2012**
(21) Application number: 07828412.2
(22) Date of filing: 26.09.2007
(51) Int. Cl.: C07F 9/09, A61K 6/083, C08F 220/58

(54) **PHOSPHATE ESTER COMPOUND, METAL SALT THEREOF, DENTAL MATERIAL, AND DENTAL COMPOSITION**
PHOSPHATESTERVERBINDUNG, METALLSALZDAVON, ZAHNMATERIAL UND DENTALMASSE
COMPOSÉ À BASE D'ESTER DE PHOSPHATE, SEL MÉTALLIQUE DE CELUI-CI, MATÉRIAU DENTAIRE ET COMPOSITION DENTAIRE

(30) Priority: 27.09.2006 JP 2006261598
(43) Date of publication of application: 13.05.2009
(73) Proprietor: Mitsui Chemicals, Inc., Tokyo 105-7117 (JP)
(72) Inventor: TAKAGI, Masatoshi, Sodegaura-shi Chiba 299-0265 (JP); OTSUJI, Atsuo, Shiga, 524-0044 (JP); NAGATOMO, Akinori, Omuta-shi Fukuoka 836-8610 (JP); SUESUGI, Kouji, Omuta-shi Fukuoka 836-8610 (JP)
(74) Representative: Wills, Andrew Jonathan
(86) International application number: PCT/JP2007/068639
(87) International publication number: WO 2008/038651

(56) References cited:
- WO-A1-2005/063778
- JP-A- 2006 176 522
- JP-A- 2007 161 622

## Description

### Technical Field

The present invention relates to a phosphate compound, a metal salt thereof, a dental material, and a dental composition.

### Background Art

Dental materials and dental compositions containing as a main component a resin composition obtained from a radical-polymerizable compound (hereinafter referred to as a polymerizable compound), such as a (meth)acrylate compound, have been put into practical use as an adhesive material, the adhesive material being used as a material for prosthetic teeth or a crown that compensates for a portion of a tooth bud or crown that has been lost due to caries of a natural tooth; a composite resin system material such as a composite resin or a bonding material that repairs by filling a damaged portion of a tooth that has occurred due to caries or the like; and a resin cement that is used to attach a prosthetic crown to a natural tooth.

Conventionally, in order to attain sufficient adhesion of dental adhesive materials to a tooth material, it has been necessary to perform a pretreatment of a tooth material such as dentine with an etching agent composed of an acidic compound such as phosphoric acid or citric acid. However, there have been problems in that dental pulp may be aggravated by the pretreatment, sufficient performances in adhesive strength of the etching agent has yet to be achieved, and the like. On the other hand, if no pretreatment with an etching agent is performed, there have been problems such as insufficient adhesion to dentine and the like.

With the aim of solving these problems, Japanese Patent Application Laid-Open (JP-A) No. 54-11149 proposes an adhesive composition containing 4-methacryloyloxyethyloxycarbonyl phthalic anhydride (4-methacryloyloxyethyl trimellitate) as a polymerizable compound, and partially-oxidized tributyl borane as a polymerization initiator. Since this composition exhibits excellent adhesiveness to a tooth material, it is used as an adhesive dental material.

Further, in order to improve adhesiveness, a methacrylate compound having a specific structure containing a phosphate group (for example, 10-methacryloyloxydecanyl phosphate or the like) has been proposed and put into practical use. However, since materials containing such a compound may undergo hydrolysis due to a highly acidic phosphate group, there is a problem with the storage stability of these materials. In order to solve this problem, JP-A No. 2003-89613 and JP-A No. 2004-131468 propose dental materials formed from a polymerizable monomer having a novel structure. However, it has been revealed that these dental materials fail to sufficiently satisfy desired performances with respect to storage stability, handleability, polymerizability, adhesiveness, and the like. JP-A No. 2006-176522 describes a polymerizable dental material containing a (meth)acrylamide phosphate. The (meth)acrylamide phosphate is said to have a high hydrolysis resistance and is said to be suitable for use as an adhesion component for self-etching dental materials such as adhesives, coating materials and composites.

Currently, in order to perform attachment of various types of restoration material (for example, metal, ceramic substance, composite resin, or the like) to a tooth material in a more simple and reliable manner, dental materials that are highly stable during storage, convenient for handling, and that exhibit higher adhesiveness and bond durability have been desired and developed.

### Disclosure of Invention

### Problem to be addressed by the invention

The present invention provides a dental material and a dental composition which address the aforementioned problems concerning dental materials and dental compositions, and exhibit excellent storage stability, handleability, polymerizability and adhesiveness. Means for addressing the problem

With a goal of addressing the aforementioned problem, the inventors have made extensive study and, as a result, arrived at the invention based on the findings thereof that the problem can be addressed with a phosphate compound having a specific structure containing an unsaturated double bond and a metal salt thereof in dental materials and dental compositions.

Namely, the means for addressing the aforementioned problem are as follows:

<1> A phosphate compound selected from the group consisting of: 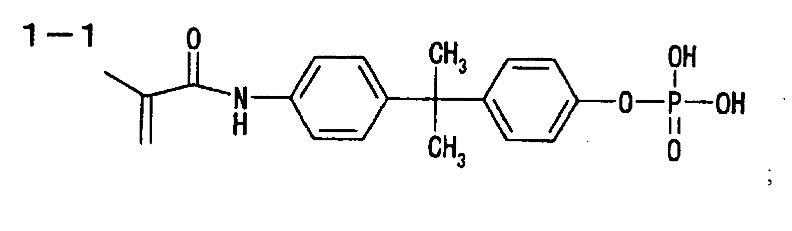 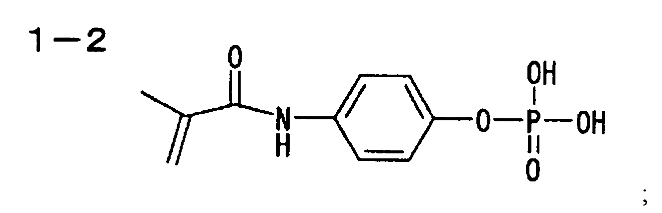 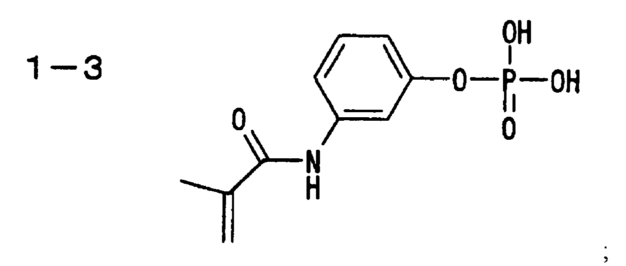 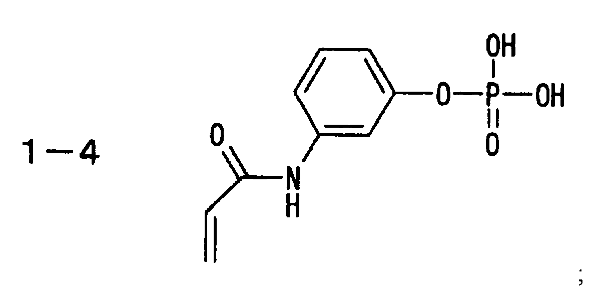 and 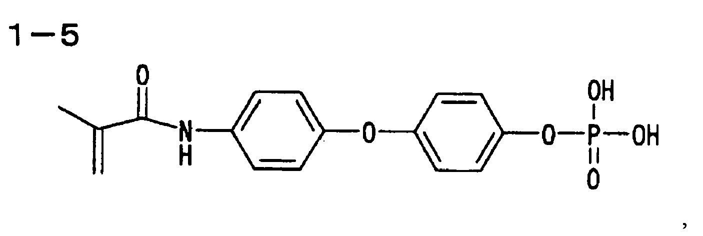 or a metal salt thereof.

<2> The phosphate compound or the metal salt of <2>, wherein the metal salt comprises a Li salt, a Na salt, a K salt, a Cu salt, an Ag salt, a Mg salt, a Ca salt, a Sr salt, a Zn salt, a Ba salt, an Al salt, a Ti salt, a Zr salt, a Sn salt, a Fe salt, a Ni salt or a Co salt.

<3> A dental material comprising a phosphate compound according to <1> or <2>, or a metal salt of the phosphate compound.

<4> The dental material of <3>, wherein the metal salt comprises a Li salt, a Na salt, a K salt, a Mg salt, a Ca salt or a Ba salt.

<5> A dental composition comprising a polymerizable compound and a polymerization initiator, the polymerizable compound comprising a phosphate compound according to <1> or <2>, or a metal salt of the phosphate compound.

<6> The dental composition of <5>, wherein the metal salt comprises a Li salt, a Na salt, a K salt, a Mg salt, a Ca salt or a Ba salt.

<7> The dental composition of <5> or <6>, further comprising a (meth)acrylate compound or a (meth)acrylic acid amide compound as a polymerizable compound.

<8> The dental composition of any of <5> to <7>, further comprising an acid group-containing monomer as a polymerizable compound.

<9> The dental composition of any of <5> to <8>, further comprising a filler.

<10> The dental composition of any of <5> to <9>, wherein the polymerization initiator comprises a heat polymerization initiator, an autopolymerization initiator or a photopolymerization initiator.

<11> A modifier for a (meth)acrylic resin, the modifier comprising a phosphate compound according to <1> or <2>, or a metal salt of the phosphate compound.

<12> A coating material comprising a phosphate compound according to <1> or <2>, or a metal salt of the phosphate compound.

<13> An adhesive material comprising a phosphate compound according to <1> or <2>, or a metal salt of the phosphate compound.

<14> A molding material comprising a phosphate compound according to <1> or <2>, or a metal salt of the phosphate compound.

### Effect of the Invention

By using the phosphate compound of the invention having a specific structure and/or a salt of the phosphate compound, it is possible to provide a dental material and a dental composition having excellent storage stability, handleability and polymerizability, being convenient for handling, and achieving even higher adhesiveness and bond durability.

### Best Mode for Carrying out the Invention

In the following, the present invention is described in further detail.

A phosphate compound of the invention contains an unsaturated double (and a metal salt of the phosphate compound) is a novel compound characterized in that it has, in its molecule, an unsaturated double bond such as a polymerizable (meth)acrylamide group and a phosphate group that is bonded to a specific aromatic ring structure.

As described above, the compound of the invention includes a phosphate group as an acid group and a group containing a nitrogen atom and an unsaturated double bond such as (meth)acrylamide group as a polymerizable group, thereby exhibiting favorable polymerizability and hydrolysis resistance (storage stability) as well.

Described herein, and useful for the understanding of the present invention, is a phosphate compound represented by formula (1). The compounds of the invention are encompassed by formula (1).

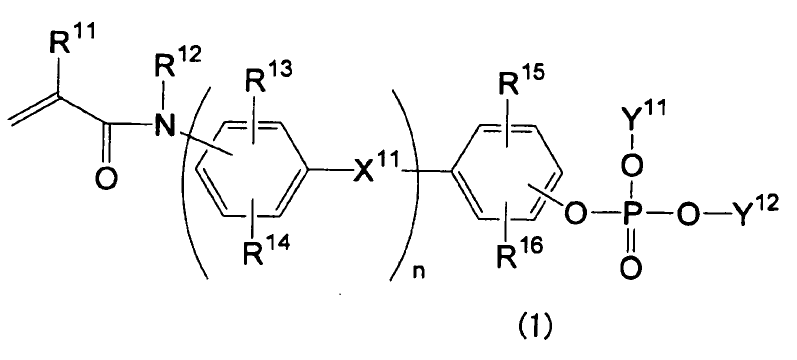

In formula (1), R¹¹ and R¹² each independently represent a hydrogen atom or an alkyl group.

In formula (1), R¹³, R¹⁴, R¹⁵ and R¹⁶ each independently represent a hydrogen atom, an alkyl group, an alkoxy group, an aralkyl group, an aryl group or a halogen atom.

In formula (1), n represents an integer of 0 or 1.

In formula (1), X¹¹ represents an -O- group, an -S- group, an -SO₂ group or a group having the following structure:

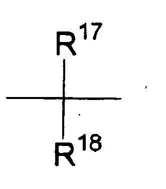

In the above group, R¹⁷ and R¹⁸ each independently represent a hydrogen atom, an alkyl group or an aryl group.

In formula (1), Y¹¹ and Y¹² independently represent a hydrogen atom or a group represented by formula (a):

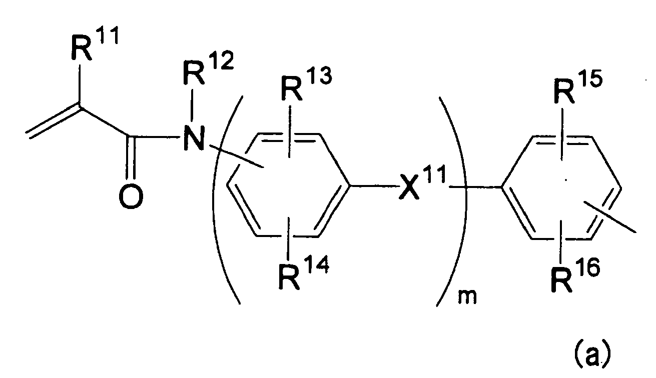

In formula (a), R¹¹ to R¹⁶ and X¹¹ have the same definitions as above, and m represents an integer of 0 or 1, wherein m is 0 when n in formula (1) is 0 and m is 1 when n is 1.

The metal salt of the phosphate compound of the invention is produced by reacting a compound of the invention with a metallic compound.

Examples of the metal salt include a Li salt, a Na salt, a K salt, a Cu salt, an Ag salt, a Mg salt, a Ca salt, a Sr salt, a Zn salt, a Ba salt, an Al salt, a Ti salt, a Zr salt, a Sn salt, a Fe salt, a Ni salt, and a Co salt. Among these metal salts, when used in dental materials or dental compositions, a Li salt, a Na salt, a K salt, a Mg salt, a Ca salt, and a Ba salt are preferable.

The phosphate compound of the invention containing a (meth)acryloyl group is synthesized according to a method in which the reaction is known per se.

Specifically, as shown in the following reaction scheme for example, the phosphate compound containing an unsaturated double bond represented by formula (1), which encompasses the compounds of the invention, is typically produced by reacting an aminophenol compound represented by formula (2-i) with a compound represented by formula (3) (for example, (meth)acryloyl chloride, (meth)acrylic acid, methyl (meth)acrylate, ethyl (meth)acrylate, propyl (meth)acrylate, or the like) to produce a compound represented by formula (2-ii), followed by reacting the compound with a known phosphoric acid derivative (for example, linear polyphosphoric acid, cyclic trimetaphosphoric acid, phosphoryl chloride, or the like) to form a phosphate.

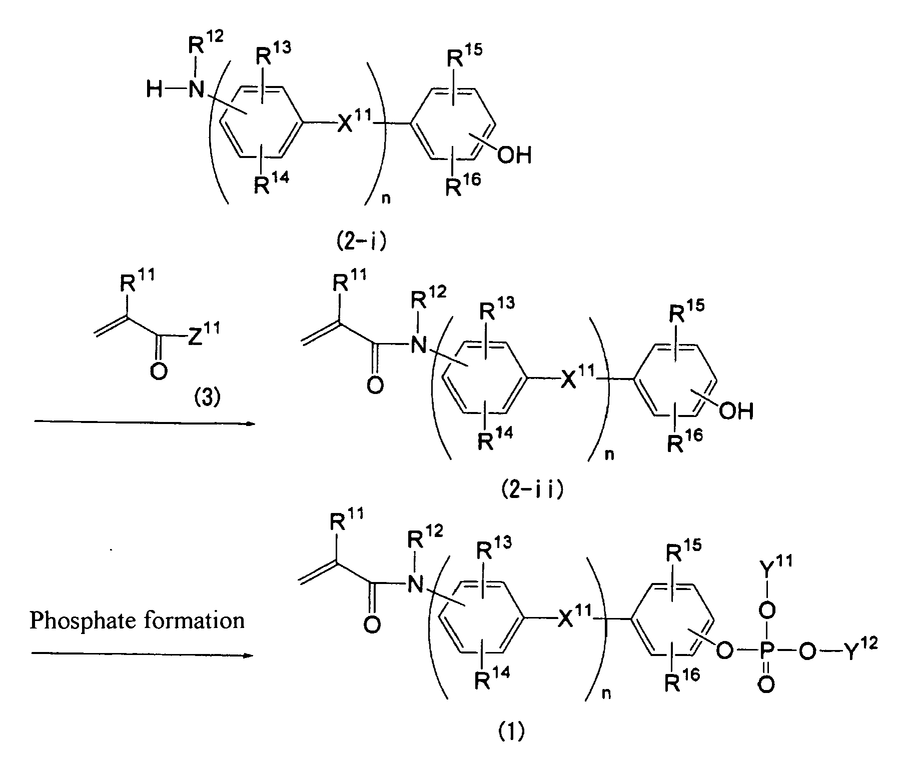

In the above scheme, R¹¹ to R¹⁶, X¹¹, n, Y¹¹ and Y¹² have the same definitions as above, and Z¹¹ is a hydroxy group, an alkoxy group having 1 to 4 carbon atoms, a chlorine atom or a bromine atom.

The aminophenol compound used as a starting compound represented by formula (2-i) can be produced by a method known in the art, such as the methods described in Journal of American Chemical Society, Vol. 68, p. 2600 (1946), UK Patent No. 1028156, JP-A Nos. 62-114942, 62-116546 and 1-172364, Pharmaceutical Bulletin, Vol. 5, p. 397 (1952), Journal of Society of Synthetic Organic Chemistry, Japan, Vol. 24, p. 44 (1966), US Patent No. 3240706, Indian Journal of Chemistry, Vol. 15B, p. 661 (1977), US Patent No. 3443943, and JP-A Nos. 5-306373 and 6-72036.

Hereinafter, the method of reacting the compound represented by formula (3) with the compound represented by formula (2-i) is described in detail.

Examples of the above method include those described in "Jikken Kagaku Koza (Experimental Chemistry Course)", 4th edition, vol. 22, p. 137 - (1992), compiled by The Chemical Society of Japan, or JP-A No. 2004-43467.

The amount of the compound represented by formula (3) to use for the reaction with the compound represented by formula (2-i) is not particularly limited, but is usually 0.1 to 100 moles, preferably 0.5 to 50 moles, more preferably 0.9 to 10 moles, with respect to 1 mole of the compound of formula (2-i).

The reaction may be conducted without a solvent, or may be conducted in an inert solvent. Examples of such a solvent include hydrocarbon solvents such as n-hexane, benzene, toluene or the like; ketone solvents such as acetone, methyl ethyl ketone, methyl isobutyl ketone or the like; ester solvents such as ethyl acetate, butyl acetate or the like; ether solvents such as diethyl ether, tetrahydrofuran, dioxane or the like; and halogen solvents such as dichloromethane, chloroform, carbon tetrachloride, 1,2-dichloroethane, perclene, or the like. These solvents may be used in combination of two or more kinds.

The reaction temperature is not particularly limited, but is usually within a range at which a reaction product does not polymerize or decompose, typically in the range of -78 to 150°C, preferably -20 to 100°C, more preferably 0 to 80°C.

The reaction time may change depending on the reaction temperature, but is usually in the range of from several minutes to 100 hours, preferably 30 minutes to 50 hours, more preferably 1 to 20 hours. It is possible to stop the reaction at an arbitrary reaction rate, by observing the reaction rate with a known analysis means (for example, liquid chromatography, thin-layer chromatography, IR, or the like).

When a (meth)acryloyl halide is used as the compound of formula (3), hydrogen halide (for example, hydrogen chloride) is generated as a byproduct. Therefore, an organic base such as triethylamine, pyridine, picoline, dimethylaniline, diethylaniline, 1,4-diazabicyclo[2.2.2]octane (DABCO) or 1,8-diazabicyclo[5.4.0]undec-7-ene (DBU), or an inorganic base such as sodium bicarbonate, sodium carbonate, potassium carbonate, lithium carbonate, sodium hydroxide, potassium hydroxide, calcium hydroxide, or magnesium oxide may be used as a dehydrohalogenation agent.

The amount of the dehydrohalogenation agent to be used is not particularly limited, but is usually 0.01 to 10 moles, preferably 0.1 to 5 moles, more preferably 0.5 to 3 moles, with respect to 1 mole of the compound represented by formula (2-i).

After the completion of the reaction, the compound as a production intermediate represented by formula (2-ii) is isolated through post-treatment according to known operation and treatment methods (for example, neutralization, solvent extraction, water washing, partition, distillation or the like). As necessary, the resulting compound represented by formula (2-ii) is separated and purified by a known method (for example, chromatography, treatment with activated carbon or adsorbent, or the like) and isolated as a highly pure compound.

Next, the method of forming a phosphate by reacting a phosphoric acid compound with the compound of formula (2-ii) is described in detail.

Examples of the method include those described in "Jikken Kagaku Koza (Experimental Chemistry Course)", 4th edition, vol. 22, p. 311 - (1992), compiled by The Chemical Society of Japan, or JP-ANo. 2001-39992.

The amount of the phosphoric acid derivative (for example, linear polyphosphoric acid, cyclic trimetaphosphoric acid, phosphoryl chloride, or the like) to react with the compound represented by formula (2-ii) is not particularly limited, but is usually 0.1 to 100 moles, preferably 0.3 to 10 moles, more preferably 0.5 to 5 moles, with respect to 1 mole of the compound of formula (2-ii).

By regulating the amount of phosphoric acid derivative used in the reaction, it is possible to selectively produce phosphoric acid monoester, phosphoric acid diester or phosphoric acid triester.

The reaction may be carried out without a solvent, or may be carried out in water or an inert solvent. Examples of the solvent include hydrocarbon solvents such as n-hexane, benzene, toluene or the like; ketone solvents such as acetone, methyl ethyl ketone, methyl isobutyl ketone or the like; ester solvents such as ethyl acetate, butyl acetate or the like; ether solvents such as diethyl ether, tetrahydrofuran, dioxane or the like; halogen solvents such as dichlolomethane, chloroform, carbon tetrachloride, 1,2-dichloloethane, perclene or the like, and other polar solvents such as acetonitrile, dimethylformamide, N,N-dimethylacetamide, m-cresol or the like. These solvents may be used in combination of two or more kinds.

The reaction temperature is not particularly limited, but is usually within the range at which a reaction product does not polymerize or decompose, such as -78 to 150°C, preferably -20 to 100°C, more preferably 0 to 80°C.

While the reaction time depends on the reaction temperature, it is usually from several minutes to 100 hours, preferably from 30 minutes to 50 hours, more preferably from 1 to 20 hours. It is possible to stop the reaction at an arbitrary reaction rate, by observing the reaction rate with a known analytical means (for example, liquid chromatography, thin-layer chromatography, IR or the like).

When phosphoryl chloride is used as the phosphoric acid derivative, it is preferable to conduct the reaction in the presence of a base and/or water. Examples of the base used herein include organic bases such as triethylamine, pyridine, picoline, dimethylaniline, diethylaniline, 1,4-diazabicyclo[2.2.2]octane (DABCO), 1,8-diazabicyclo[5.4.0]undec-7-ene (DBU) and the like, or inorganic bases such as sodium bicarbonate, sodium carbonate, potassium carbonate, lithium carbonate, sodium hydroxide, potassium hydroxide, calcium hydroxide, magnesium oxide and the like.

The amount of the base is not particularly limited, but is usually 0.1 to 100 moles, preferably 0.1 to 10 moles, more preferably from 0.1 to 5 moles, with respect to 1 mole of the compound represented by formula (2-ii).

Other than by regulating the amount of phosphoric acid derivative, as described above, it is also possible to selectively produce phosphoric acid monoester, phosphoric acid diester or phosphoric acid triester, which are included in the compound of the invention by regulating the reaction conditions such as temperature, reaction time, type or amount of organic solvent, type or amount of base, or the like.

In the production of the phosphate compound of the invention containing an unsaturated double bond compound of the invention or a metal salt of the phosphate compound, it is preferable to use a polymerization inhibitor in order to inhibit polymerization of the product during or after the reaction. Examples of the polymerization inhibitor include various known compounds such as 4-methoxyphenol, hydroquinone, phenothiazine and the like. The amount of polymerization inhibitor to be used is not particularly limited, but is usually 0.01 to 5% by weight, preferably 0.05 to 3% by weight, with respect to the raw material mixture or the reaction product in the reactive system.

After the completion of the reaction, the phosphate compound of the invention containing an unsaturated double bond compound of the invention is isolated through post-treatment according to known operation and treatment methods (for example, neutralization, solvent extraction, water washing, partition, distillation or the like). As necessary, the resulting phosphate compound is separated and purified by a known method (for example, chromatography, treatment with activated carbon or various absorbents, and the like) and isolated as a highly pure compound.

It is preferable that the compound is subjected to filtration as a solution so as to include impurities such as insoluble substances, insoluble particles, wastes, dust, foreign substances or the like at a low content to exhibit high transparency. For example, the impurities can be removed by filtering the phosphate compound of the invention containing an unsaturated double bond using a filter in a clean room or the like.

Further, as necessary, by subjecting the intermediate product to the operation or treatment method as described above in the production of the phosphate compound containing an unsaturated double bond represented by formula (1), the purity can be even more heightened.

The metal salt of the phosphate compound of the invention containing an unsaturated double bond can be produced by reacting the thus obtained phosphate compound containing an unsaturated double bond with a metal compound of various kinds by a known method of producing a metal phosphate.

Some compounds having, as an unsaturated double bond, a (meth)acryloyl group and a phosphoric acid ester group in one molecule are known. For example, JP-A Nos. 2003-89613 and 2004-131468 disclose a dental material containing a phosphate compound having a (meth)acryloyl group. However, this dental material is not considered to have sufficient performances that are necessary for a dental adhesive, such as storage stability, handleability, polymerizability, adhesiveness or bond durability.

It is an unexpected surprising result that the dental material containing a phosphate compound having a specific structure according to the invention exhibits excellent performances in handleability, polymerizability, adhesiveness and bond durability. Accordingly, the invention provides a useful dental material that can never be achieved before.

The dental material of the invention includes, as an organic material component of the dental material, at least one of the phosphate compounds of the invention containing an unsaturated double bond and/or a metal salt of the phosphate compound.

The dental material of the invention encompasses general organic dental materials widely used in dental therapy including the dental composition as described below, and examples of such dental materials include crown materials used for a crown or prosthetic tooth, dental filling materials such as composite resin, root canal filler and bonding material, dental adhesives/dental luting agents such as resin cement and orthodontic adhesive, Fischer sealant, coating material, crown/bridge/inlay resin, dental abutment construction material, denture base resin, and denture base repair resin.

### <Dental composition>

The dental composition of the invention includes, in addition to a polymerizable compound, a polymerization initiator as an essential component, which encompasses a polymerizable composition before the initiation of polymerization and curing and a cured product obtained by polymerizing and curing the polymerizable composition.

The dental composition of the invention is a polymerizable composition including a polymerizable compound and a polymerization initiator as an essential component, and the polymerizable compound includes a phosphate compound of the invention containing an unsaturated double bond and/or a metal salt of the phosphate compound.

### (Polymerizable compound)

In the dental composition of the invention containing a phosphate compound of the invention containing an unsaturated double bond and/or a metal salt of the phosphate compound, a single kind of the compound of the invention and/or a metal salt of the phosphate compound may be used, or two or more kinds of the compound of the invention may be used in combination.

The phosphate compound of the invention and/or a metal salt of the phosphate compound is usually used at an amount in the range of 1 to 100% by mass in 100 parts by mass of the total amount of polymerizable compound in the dental composition of the invention.

The dental composition of the invention may contain, as a polymerizable compound as one of the essential component, a polymerizable compound other than the phosphate compound of the invention and/or a metal salt of the phosphate compound in such a range that the desired effects of the invention is not impaired, in addition to the phosphate compound of the invention and/or a metal salt of the phosphate compound.

The above polymerizable composition is not particularly limited, and a wide variety of known polymerizable compounds (polymerizable monomers or polymerizable oligomers) that are used in the field of dental material may be used.

In consideration of polymerizability and curability, the polymerizable compound other than the phosphate compound of the invention and/or a metal salt of the phosphate compound is preferably a (meth)acrylate compound or a (meth)acrylic acid amide compound.

Among these, (meth)acrylate compounds, which are less toxic, rapidly polymerized, hardly undergoes hydrolysis and relatively easily produced, are more preferable.

Examples of the (meth)acrylate compound include monofunctional or multifunctional (meth)acrylate compounds, for example, alkyl (meth)acrylate compounds such as methyl (meth)acrylate, ethyl (meth)acrylate, butyl (meth)acrylate, hexyl (meth)acrylate, 2-ethylhexyl (meth)acrylate, dodecyl (meth)acrylate, lauryl (meth)acrylate, cyclohexyl (meth)acrylate, benzyl (meth)acrylate, isobomyl (meth)acrylate, adamantyl (meth)acrylate and the like; hydroxyalkyl (meth)acrylate compounds such as 2-hydroxyethyl (meth)acrylate, 2- or 3-hydroxypropyl (meth)acrylate, 4-hydroxybutyl (meth)acrylate, 5-hydroxypentyl (meth)acrylate, 6-hydroxyhexyl (meth)acrylate, 1,2- or 1,3-dihydroxypropyl mono(meth)acrylate, erythritol mono(meth)acrylate and the like; polyethylene glycol mono(meth)acrylate compounds such as diethylene glycol mono(meth)acrylate, triethylene glycol mono(meth)acrylate, polyethylene glycol mono(meth)acrylate, polypropylene glycol mono(meth)acrylate and the like; (poly)glycol monoalkyl ether (meth)acrylates such as ethylene glycol monomethyl ether (meth)acrylate, ethylene glycol monoethyl ether (meth)acrylate, diethylene glycol monomethyl ether (meth)acrylate, triethylene glycol monomethyl ether (meth)acrylate, polyethylene glycol monomethyl ether (meth)acrylate, polypropylene glycol monoalkyl ether (meth)acrylate and the like; fluoroalkyl (meth)acrylate compounds such as perfluorooctyl (meth)acrylate, hexafluorobutyl (meth)acrylate and the like; silane compounds having a (meth)acryloxyalkyl group such as γ-(meth)acryloxypropyltrimethoxysilane, γ-(meth)acryloxypropyltri(trimethylsiloxy)silane and the like; carboxylic acid-containing (meth)acrylate compounds such as β-methacryloyloxyethyl hydrogen phthalate, β-methacryloyloxyethyl hydrogen succinate, β-methacryloyloxyethyl maleate and the like; halogen-containing (meth)acrylates such as 3-chloro-2-hydroxypropyl methacrylate and the like; and (meth)acrylate compounds having a heterocyclic ring, such as tetrahydrofurfuryl (meth)acrylate and the like; alkanepolyol poly(meth)acrylate compounds such as ethylene glycol di(meth)acrylate, propylene glycol di(meth)acrylate, butylene glycol di(meth)acrylate, neopentyl glycol di(meth)acrylate, hexylene glycol di(meth)acrylate, trimethylolpropane trim(meth)acrylate, pentaerythritol tetra(meth)acrylate and the like; polyoxyalkanepolyol poly(meth)acrylate compounds such as diethylene glycol di(meth)acrylate, triethylene glycol di(meth)acrylate, polyethylene glycol di(meth)acrylate, dipropylene glycol di(meth)acrylate, polypropylene glycol di(meth)acrylate, dibutylene glycol di(meth)acrylate, dipentaerythritol hexa(meth)acrylate and the like;

aliphatic, alicyclic or aromatic (meth)acrylate compounds represented by the following formula (4)

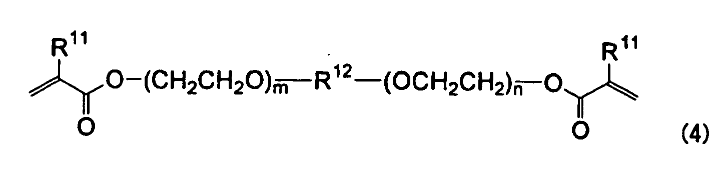

(in the above formula, R¹¹ represents a hydrogen atom or a methyl group, m and n each represent 0 or a positive integer, and R¹² represents a divalent organic linking group);
alicyclic or aromatic epoxy di(meth)acrylates represented by the following formula (5)

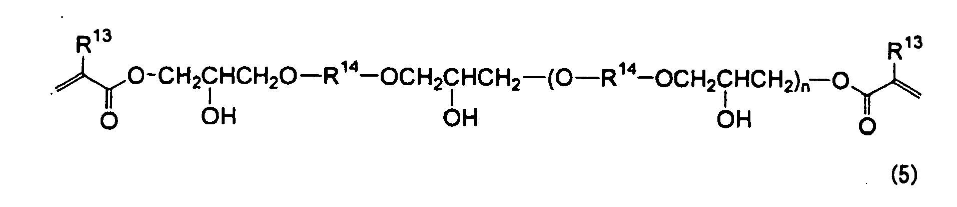

(in the above formula, R¹³ represents a hydrogen atom or a methyl group, n represents 0 or a positive integer, and R¹⁴ represents a divalent organic linking group); and (meth)acrylate compounds having a urethane bond in a molecule represented by the following formula (6)

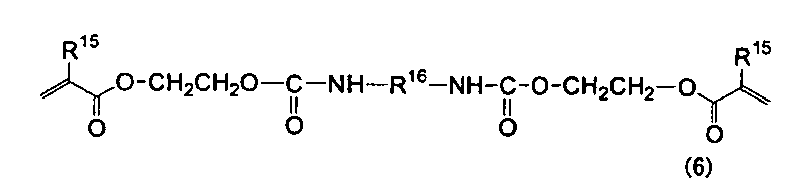

(in the above formula, R¹⁵ represents a hydrogen atom or a methyl group, and R¹⁶ represents a divalent organic linking group).

In formulae (4) and (5), the divalent organic linking groups R¹² and R¹⁴ each represent -(CH₂)₂-, -(CH₂)₄-, -(CH₂)₆-, or

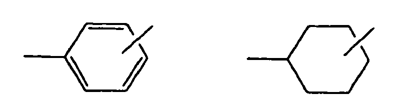

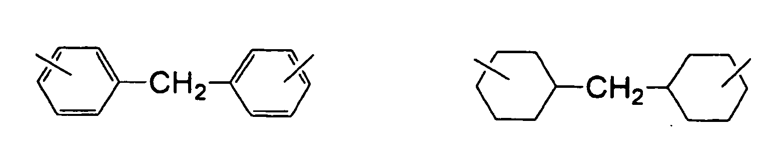

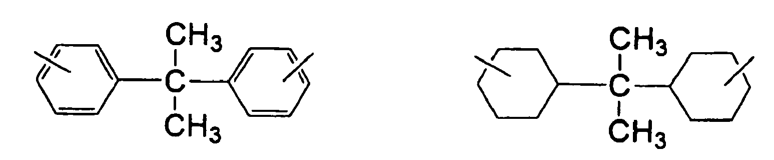

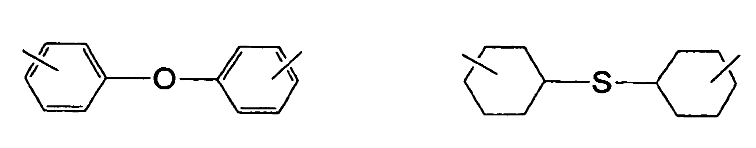

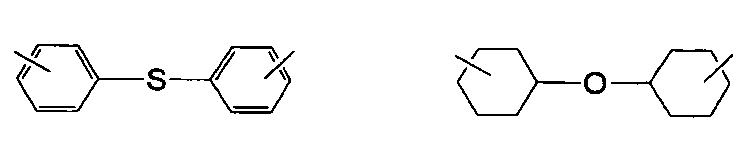

any of the above structures.

In formula (6), the divalent organic linking group R¹⁶ represents -(CH₂)₂-, -(CH₂)₄-, -(CH₂)₆-, or

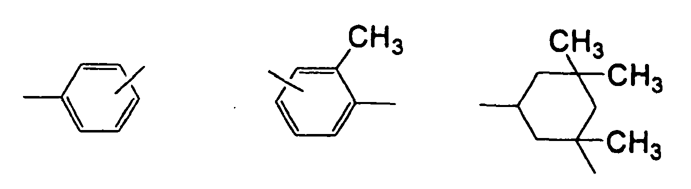

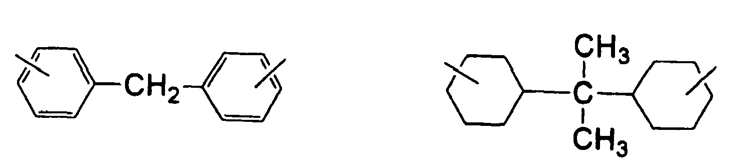

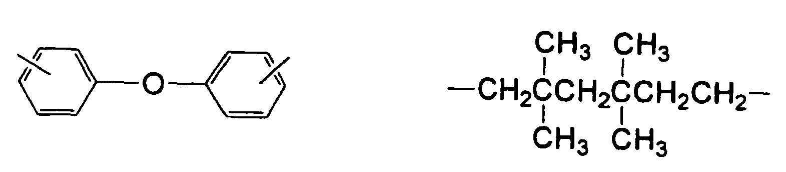

any of the above structures.

Among the above-illustrated polymerizable compounds, methyl methacrylate, ethyl methacrylate, 2-hydroxyethyl methacrylate, diethylene glycol monomethyl ether methacrylate, tetraethylene glycol monomethyl ether methacrylate, ethylene glycol dimethacrylate, triethylene glycol dimethacrylate, and the compounds represented by the following formulae (7), (8) and (9) are more preferable.

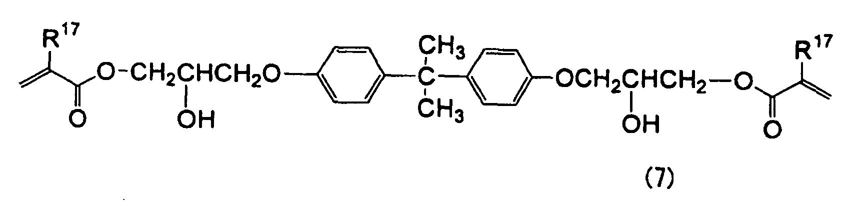

(in the above formula, R¹⁷ represents a hydrogen atom or a methyl group)

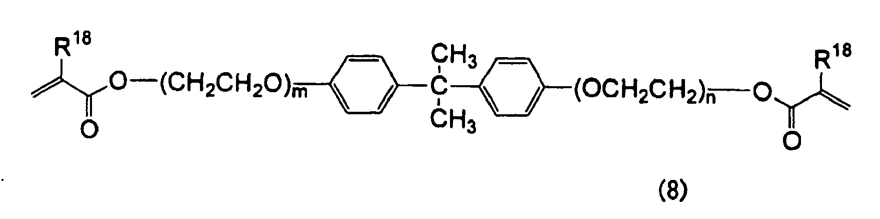

(in the above formula, R¹⁸ represents a hydrogen atom or a methyl group, and m+n is 2.6 on average)

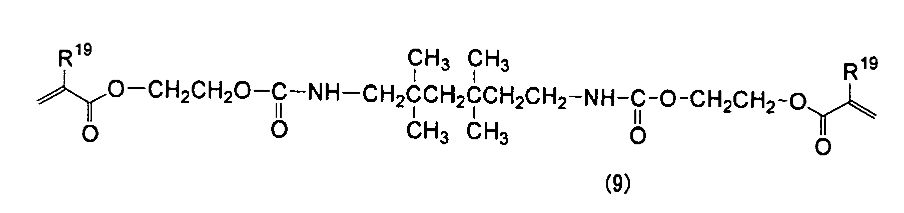

(in the above formula, R¹⁹ represents a hydrogen atom or a methyl group)

The polymerizable compound other than the phosphate compound represented by formula (1) and/or a metal salt of the phosphate compound is normally used at an amount in the range of 5 to 90% by mass, preferably 5 to 80% by mass, more preferably 10 to 70% by mass, still more preferably 10 to 50% by mass, of 100 parts by mass of all polymerizable compounds contained in the dental composition of the invention.

The dental composition of the invention may contain a known acid group-containing monomer or the like, other than the phosphate compound of the invention and/or a metal salt of the phosphate compound, within such a range that the desired effects of the invention are not impaired. Preferable examples of the acid group include a carboxyl group, a phosphoric group, and a sulfonic group.

Among the acid group-containing polymerizable monomers, examples of the monomer having at least one carboxyl group in one molecule include monocarboxylic acids, dicarboxylic acids, tricarboxylic acids, tetracarboxylic acids, and their derivatives.

Specific examples of the acid group-containing polymerizable monomer include (meth)acrylic acid, maleic acid, p-vinylbenzoic acid, 11-(meth)acryloyloxy-1,1-undecanedicarboxylic acid (MAC-10), 1,4-di(meth)acryloyloxyethylpyromellitic acid, 6-(meth)acryloyloxyethylnaphthalene-1,2,6-tricarboxylic acid, 4-(meth)acryloyloxymethyltrimellitic acid and an anhydride thereof, 4-(meth)acryloyloxyethyltrimellitic acid and an anhydride thereof, 4-(meth)acryloyloxybutyltrimellitic acid and an anhydride thereof, 4-[2-hydroxy-3-(meth)acryloyloxy]butyltrimellitic acid and an anhydride thereof, 2,3-bis(3,4-dicarboxybenzoyloxy)propyl(meth)acrylate, N-o-di(meth)acryloyloxytyrosine, o-(meth)acryloyloxytyrosine, N-(meth)acryloyloxytyrosine, N-(meth)acryloyloxyphenylalanine, N-(meth)acryloyl-p-aminobenzoic acid, N-(meth)acryloyl-o-aminobenzoic acid, N-(meth)acryloyl-5-aminosalicylic acid, N-(meth)acryloyl-4-aminosalicylic acid, 2-, 3- or 4-(meth)acryloyloxybenzoic acid, an addition product formed from 2-hydroxyethyl (meth)acrylate and pyromellitic dianhydride (PMDM), an addition product formed from 2-hydroxyethyl (meth)acrylate and maleic anhydride, an addition product formed from 2-hydroxyethyl (meth)acrylate and 3,3',4,4'-benzophenonetetracarboxylic dianhydride (BTDA), an addition product formed from 2-hydroxyethyl (meth)acrylate and 3,3',4,4'-biphenyltetracarboxylic dianhydride, 2-(3,4-dicarboxybenzoyloxy)-1,3-di(meth)acryloyloxypropane, an adduct formed from N-phenylglycine or N-tolylglycine and glycidyl (meth)acrylate, 4-[(2-hydroxy-3-(meth)acryloyloxypropyl)amino]phthalic acid, 3- or 4-[N-methyl-N-(2-hydroxy-3-(meth)acryloyloxypropyl)amino]phthalic acid, and the like.

Examples of the monomer having at least one phosphoric group in one molecule include 2-(meth)acryloyloxyethyl acid phosphate, 2- or 3-(meth)acryloyloxypropyl acid phosphate, 4-(meth)acryloyloxybutyl acid phosphate, 6-(meth)acryloyloxyhexyl acid phosphate, 8-(meth)acryloyloxyoctyl acid phosphate, 10-(meth)acryloyloxydecyl acid phosphate, 12-(meth)acryloyloxydodecyl acid phosphate, bis{2-(meth)acryloyloxyethyl}acid phosphate, bis{2- or 3-(meth)acryloyloxypropyl}acid phosphate, 2-(meth)acryloyloxyethylphenyl acid phosphate, 2-(meth)acryloyloxyethyl-p-methoxyphenyl acid phosphate, and the like. In the above compounds, the phosphoric group may be replaced by a thiophosphoric group.

Examples of the monomers having at least one sulfonic group in one molecule include 2-sulfoethyl (meth)acrylate, 2-sulfo-1-propyl (meth)acrylate, 1-sulfo-2-propyl (meth)acrylate, 1-sulfo-2-butyl (meth)acrylate, 3-sulfo-2-butyl (meth)acrylate, 3-bromo-2-sulfo-2-propyl (meth)acrylate, 3-methoxy-1-sulfo-2-propyl (meth)acrylate, 1,1-dimethyl-2-sulfoethyl (meth)acrylamide, and the like.

The acid group-containing monomer as mentioned above is normally used at an amount within the range of 0.01 to 100 parts by mass, preferably 0.1 to 50 parts by mass, more preferably 0.5 to 20 parts by mass, still more preferably 1 to 10 parts by mass, with respect to 100 parts by mass of all polymerizable compounds in the dental composition of the invention.

The viscosity of the polymerizable compound as a whole formed from the phosphate compound represented by formula (1) and/or a metal salt of the phosphate compound and other polymerizable compound is not particularly limited, but is usually 10 to 1,000,000 cP (mPa·s), preferably 100 to 1,000,000 cP (mPa·s) from the viewpoint of handleability upon mixing, more preferably 100 to 100,000 cP (mPa·s).

The total content of the polymerizable compound, which is an essential component in the dental composition of the invention, is preferably in the range of 5 to 50% by weight, more preferably 10 to 30% by weight, of the total weight of the dental composition.

### (Polymerization initiator)

The polymerization initiator that is used in the dental composition of the invention is not particularly limited, and known polymerization initiator of various kinds (for example, heat-polymerization initiators, autopolymerization initiators, photopolymerization initiators or the like) may be suitably used.

The heat-polymerization initiators include, for example, organic peroxides, diazo type compounds, and the like. Examples of the organic peroxide include diacyl peroxides such as diacetyl peroxide, diisobutyl peroxide, didecanoyl peroxide, benzoyl peroxide, succinic acid peroxide and the like; peroxydicarbontes such as diisopropyl peroxydicarbonate, di-2-ethylhexyl peroxydicarbonate, diallyl peroxydicarbonate and the like; peroxy esters such as tert-butyl peroxyisobutyrate, tert-butyl neodecanate, cumene peroxyneodecanate and the like; sulfonyl peroxides such as acetylcyclohexylsulfonyl peroxide and the like.

Examples of the diazo type compound include 2,2'-azobisisobutyronitrile, 4,4'-azobis(4-cyanovaleric acid), 2,2'-azobis(4-methoxy-2,4-dimethoxyvaleronitrile), 2,2'-azobis(2-cyclopropylpropionitrile) and the like.

In view of the advantages that polymerization can be conducted in a short time or the like, compounds having a decomposition half-life at 80°C of not more than 10 hours are preferable, and among the above compounds, benzoyl peroxide and 2,2'-azobisisobutyronitrile are more preferable compounds.

Specific examples of the autopolymerization initiators include so-called redox polymerization initiators that are a combination of heat-polymerization initiator and reducing compound that start polymerization at ordinary temperature upon reaction, and trialkyl boron derivatives that can polymerize by themselves.

The redox polymerization initiator is not particularly limited, and known heat-polymerization initiator and reducing compound may be used. Examples of the reducing compound used in the redox polymerization initiator include organic or inorganic compounds, including amine compounds such as N,N-dimethylaniline, p-N,N-dihydroxyethylaniline, N,N-dibenzylaniline, N,N-dimethyl-p-toluidine, N,N-diethyl-p-toluidine, N,N-dihydroxy-p-toluidine, N,N-dimethyl-p-tert-butylaniline, N,N-dimethylanisidine, N,N-diethyl-p-chloroaniline, p-N,N-dimethylamino benzoic acid, methyl p-N,N-dimethylaminobenzoate, methyl p-N,N-diethylaminobenzoate, p-N,N-diethylaminobenzoic acid, ethyl p-N,N-dimethylaminobenzoate, ethyl p-N,N-diethylaminobenzoate, 2-n-butoxyethyl p-N,N-dimethylaminobenzoate, 2-n-butoxyethyl p-N,N-diethylaminobenzoate, p-N,N-dimethylamino benzaldehyde, p-N,N-dimethylaminobenzonitrile, p-N,N-diethylaminobenzonitrile, p-dimethylaminophenethyl alcohol, N,N-dimethylaminoethyl methacrylate, triethylamine, tributylamine, tripropylamine, N-ethyl ethanolamine, N-phenylglycine, N-tolylglycine, N-(3-methacryloyloxy-2-hydroxypropyl)phenyl glycine, and the like; aromatic sulfinic acids such as benzenesulfinic acid, o-toluenesulfinic acid, p-toluenesulfinic acid, ethylbenzenesulfinic acid, decylbenzenesulfinic acid, dodecylbenzenesulfinic acid, chlorobenzenesulfinic acid and the like, or salts thereof; barbituric acid derivatives such as 5-butylaminobarbituric acid, 1-benzyl-5-phenylbarbituric acid and the like; sulfurous acid, bisulfurous acid, metabisulfurous acid, pyrosulfurous acid, thiosulfuric acid, dithionous acid, and salts thereof (for example, a sodium salt, a potassium salt, a sodium hydrogen salt, a potassium hydrogen salt, or the like).

Examples of the autopolymerization initiator that can polymerize by itself include tripropyl boron, triisopropyl boron, tri-n-butyl boron, tri-n-amyl boron, triisoamyl boron, tri-sec-amyl boron, and trialkyl boron oxides obtained by partially oxidizing these compounds.

The photopolymerization initiator is a compound that starts polymerization upon being excited by irradiation with visible light or ultraviolet light, and examples thereof include α-diketone compounds, phosphorus atom-containing compounds and the like, such as benzil, camphorquinone, α-naphthyl, p,p'-dimethoxybenzil, pentadione, 1,4-phenanthrenequinone, naphthoquinone, trimethylbenzoyldiphenylphosphine oxide and the like.

These compounds may be used singly or in combination of two or more kinds.

When a photopolymerization initiator is used as the polymerization initiator, it is preferable to use a photopolymerization accelerator in combination for the purpose of improving polymerizability.

As the photopolymerization accelerator, the aforementioned reducing compounds that can be used in the redox polymerization initiators (such as amine compounds, aromatic sulfinic acid or salts thereof, barbituric acid derivatives or the like) may be used.

When used as the photopolymerization accelerator, these compounds may be used singly or in combination of two or more kinds.

Among these compounds, ethyl p-N,N-dimethylaminobenzoate, methyl p-N,N-dimethylaminobenzoate, 2-n-butoxyethyl p-N,N-dimethylaminobenzoate, N,N-dimethylaminoethyl methacrylate, and the like are preferably used as the photopolymerization accelerator.

Among the combinations of a photopolymerization initiator and a photopolymerization accelerator, a combination of camphorquinone or trimethylbenzoyldiphenylphosphine oxide as the photopolymerization initiator and ethyl p-N,N-dimethylaminobenzoate or 2-n-butoxyethyl p-N,N-dimethylaminobenzoate as the photopolymerization accelerator is more preferable.

The amount of these polymerization initiators is not particularly limited, but is usually 0.001 to 10 parts by mass, preferably 0.001 to 5 parts by mass, even more preferably 0.005 to 2 parts by mass, with respect to 100 parts by mass of the polymerizable compound.

### (Filler)

The dental composition of the invention may further include a filler for the purpose of ensuring mechanical strength and imparting X-ray contrast properties.

The fillers that may be used in the invention are not particularly limited, and known inorganic or organic fillers are usually used.

Examples of the inorganic filler include elements in the I, II, III and IV groups in the periodic table, transition metals, and oxides, chlorides, sulfites, carbonates, phosphates or silicates thereof, or mixtures thereof. Specific examples include glass powders of silicon dioxide, lanthanum glass, barium glass, and strontium glass and the like; quartz powder; glass fillers containing barium sulfate, aluminum oxide, titanium oxide, barium salts, glass beads, glass fiber, barium fluoride, lead salts, or talc; and silica gel, colloidal silica, zirconium oxide, tin oxide, carbon fiber, and other ceramic powder. Among the inorganic fillers, examples of cation-eluting fillers include inorganic compounds, including hydroxides such as calcium hydroxide and strontium hydroxide and oxides such as zinc oxide, silicate glass, and fluoroaluminosilicate glass.

Examples of the organic filler include polymethyl methacrylate, polyethyl methacrylate, polymethyl methacrylate-polyethyl methacrylate copolymers, crosslinked polymethyl methacrylate, crosslinked polyethyl methacrylate, ethylene-vinyl acetate copolymers, styrene-butadiene copolymers, acrylonitrile-styrene copolymers, and acrylonitrile-styrene-butadiene copolymers. These organic fillers may be used singly or in combination of two or more thereof.

Organic-inorganic composite fillers, which are obtained by adding a polymerizable compound to the inorganic filler to form a paste and then polymerizing and pulverizing the paste, may also be suitably used in the invention.

Among these fillers, inorganic fillers are preferable, and glass powders obtained by finely pulverizing the aforementioned glasses are more preferable.

In general, since it is important that the presence of the filler is clearly determined in an X-ray photograph, the filler used in the invention preferably has X-ray contrast properties. In order to impart X-ray contrast properties to the glass powder, an element having X-ray contrast properties (heavy metal element) such as barium, strontium, zirconium, bismuth, tungsten, germanium, molybdenum, lanthanide or the like is usually added as a component of the glass.

The particle size or shape of the filler is not particularly limited, and the average particle size of the filler is usually 0.01 to 100 µm, preferably 0.01 to 50 µm, more preferably 0.01 to 10 µm, even more preferably 0.1 to 3 µm.

The refractive index of the filler is in the range of 1.53 to 1.67, particularly preferably in the range of 1.54 to 1.65.

In the dental composition of the invention, it is preferable that the formulation of polymerizable compound is adjusted or the type of filler is selected so that the difference between the refractive index of the filler and the refractive index of a cured product (resin matrix) of the polymerizable compound is 0.05 or less, more preferably 0.02 or less.

The content of the fillers in the dental composition of the invention is usually 5 to 2000 parts by mass, preferably 100 to 1000 parts by mass, more preferably 100 to 700 parts by mass, with respect to 100 parts by mass of the polymerizable compound.

The dental composition of the invention contains, as an essential component, a polymerizable compound including a phosphate compound represented by formula (1) and/or a metal salt of the phosphate compound, a polymerization initiator, and optionally a filler. Additionally, the dental composition of the invention may further contain a wide variety of known additives as long as the desired effects are not impaired. Examples of such additives include a coloring agent, a pigment, a dye, a stabilizer, a polymer powder, a UV absorber, a polymerization initiator, an antioxidant, a solvent (for example, an organic solvent such as hexane, heptane, octane, toluene, dichloromethane, methanol, ethanol or ethyl acetate, water, and the like), a thickener (for example, polyvinyl pyrrolidone, carboxymethyl cellulose, polyvinyl alcohol, and the like), a sterilizing agent, a disinfectant, a stabilizer, a preservative, and the like.

The method of producing the dental composition of the invention is not particularly limited, and can be suitably carried out according to a known method. One example of the method, in the case of a dental repairing composite resin, is a method in which predetermined amounts of polymerizable compound, polymerization initiator, filler and optional additives are weighed out, and then mixed and kneaded to prepare a composition in the form of a paste.

In order to remove insolubles or foreign substances before use, the polymerizable compound is preferably purified by a process such as filtration, prior to conducting the polymerization. Further, it is preferable that the composition is sufficiently subjected to degassing or defoaming under reduced pressure in order to prevent formation of bubbles in the cured product thereof.

Examples of the method of using the dental composition of the invention include, in the case of a dental repairing composite resin, a method of filling a cavity of the patient directly with the composition in the form of a paste prepared by the method described above and curing it by irradiating with light used in dental therapy; or when the tooth crown to be formed is an inlay or a crown, a method of applying the composition in the form of a paste onto a model of a cavity tooth or an abutment tooth that has been prepared from an imprint of the mouth of the patient so as to reproduce the shape of the tooth crown, and then curing it by irradiating with active light in a similar manner to the above.

The curing or polymerization of the dental composition of the invention can be preferably carried out by irradiation with active light such as ultraviolet light or visible light. Examples of the source of active light include a fluorescent lamp, various kinds of mercury lamps, a xenon lamp, a tungsten lamp, a halogen lamp, sunlight and the like.

The time for irradiation with active light is not particularly limited due to the influences of the wavelength or intensity of active light, the shape of the dental composition that has been filled, or the like, but is usually 1 second to 5 minutes. The temperature at the time of polymerization or curing is usually in the range of 0 to 100°C, preferably 5 to 60°C. In consideration of convenience in dental therapy and influences or impacts on the patient, the composition may be formulated so that the polymerization or curing can be completed in a short time, particularly within 1 to 30 minutes, at about ordinary temperatures (20 to 40°C).

Further, in the method of manufacturing a rigid methacrylic resin-based prosthetic tooth, the polymerizable compound, polymerization initiator and filler are weighed out in predetermined amounts, optionally compounded with a coloring agent, a pigment or the like, and kneaded uniformly to prepare a composition in the form of a paste. Subsequently, the composition is inserted into a prosthetic tooth mold and compression-molded. Namely, for example, the composition is polymerized and molded by heating it under pressure in the mold. As the polymerization catalyst, the heat polymerization initiator described above can be preferably used. After the completion of polymerization, the molded product is removed from the mold, thereby obtaining a prosthetic tooth.

Hereinafter, the invention is described more specifically with reference to the following Examples, but the invention is not limited to these Examples.

### Synthesis Example 1

### <Production of unsaturated double bond-containing compound 2-ii-1 >

168.2 g (0.74 mole) of 2-(4'-aminophenyl)-2-(4'-hydroxyphenyl) propane and 350 g of N,N-dimethylacetamide were mixed and dissolved to produce a solution. To the solution, 77.0 g (0.74 mole) of methacrylic acid chloride was added dropwise at 40°C over a period of 2 hours. After heating the solution at 50°C for another 1 hour, it was confirmed by liquid chromatography that the reaction had been almost completed. The solution was then cooled to room temperature. The solution was diluted with 300 ml ethyl acetate, then washed and partitioned until the aqueous phase became neutral, and thereafter the organic phase was recovered. The organic phase was concentrated by distilling away the solvent under reduced pressure to precipitate a solid, and the solid was collected by filtration. The solid was then sludge-purified with a mixed solvent of methanol/water to give 180.7 g (0.62 mole) of 2-(4'-methacryloylaminophenyl)-2-(4'-hydroxyphenyl) propane (compound of formula (2-ii-1) below) in the form of a colorless powdery crystal.
Yield: 82%, purity (HPLC area percentage): 99%.

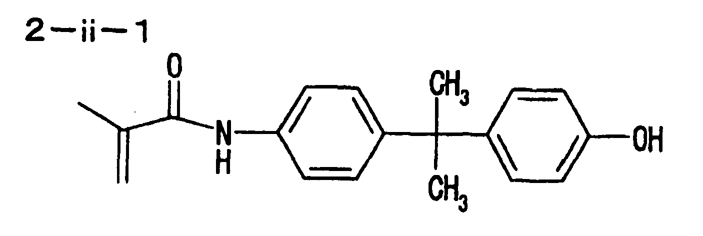

### <Production of phosphate compound 1-1>

73.6 g (0.48 mole) of phosphoryl chloride and 100 ml tetrahydrofuran were weighed out and mixed to produce a solution, and this solution was cooled to -5°C. To the solution, a mixed solution containing 118.2 g (0.40 mole) of the compound produced in Synthesis Example 1, 48.6 g (0.48 mole) of triethylamine and 200 ml tetrahydrofuran was added dropwise over a period of 2 hours. The mixture was stirred for 1 hour at the same temperature, and disappearance of the starting materials was confirmed by liquid chromatography. Subsequently, the reaction solution was subjected to hydrolysis while adding dropwise a mixture of 25.2 g (1.4 moles) of water, 80.9 g (0.8 mole) of triethylamine and 5.7 g of tetrahydrofuran, at 0°C.

The reaction solution was subjected to extraction with 300 ml n-butanol, then washed with water and partitioned to collect the organic phase. The solvent was distilled away from the organic phase under reduced pressure, thereby obtaining 148.2 g of 4-[1'-(4"-methacryloylaminophenyl)-1'-methylethyl]phenyl phosphate (compound of formula (1-1) below) in the form of a slightly yellow transparent viscous liquid.
Yield: 90%, purity (HPLC area percentage): 92%.

Spectral data and results of mass analysis of the compound are as follows:
H¹-NMR δ (DMSO-d6): 1.58 (s, 6H), 1.92 (s, 3H), 2.05 (s, 2H), 5.46 (m, 1H), 5.78 (m, 1H), 7.00-7.20 (m, 6H), 7.50-7.60 (d, 2H), 8.00-8.05 (br, 1H)
IR (cm⁻¹): 1661, 1219, 971
FD-MS (m/e): 376 (M+1), 375 (M), 295 (M-80)

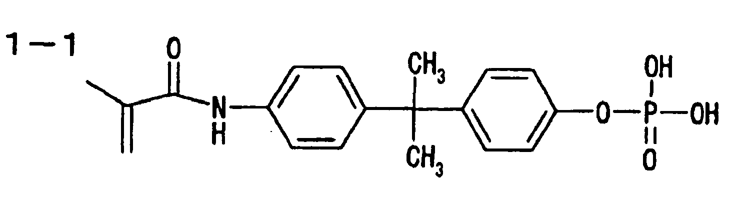

### Synthesis Example 2

### <Production of unsaturated double bond-containing compound 2-ii-2>

32.7 g (0.30 mole) of p-aminophenol and 80 g of N,N-dimethylacetamide were mixed and dissolved to produce a solution. To the solution, 29.8 g (0.285 mole) of methacrylic acid chloride was added dropwise at 50°C over a period of 2 hours. After heating the solution at 50°C for another 2 hours, it was confirmed by liquid chromatography that the reaction had been almost completed. The solution was then cooled to room temperature. The solution was washed and partitioned with 750 g of ethyl acetate and 1300 g of distilled water, and the organic phase was washed with NaHCO₃ water. Thereafter, 200 g of toluene was added to the organic phase, and the solvent was distilled away under reduced pressure. Concentration of the organic phase was conducted while further adding 400 g of toluene in twice. The concentration was completed when a solid precipitated. The resulting solid was subjected to sludging with 500 g of toluene, then filtered and washed to give a moistened material. It was dried at 40°C in a nitrogen stream, thereby obtain 36.7 g (0.207 mole) of 4-methacryloylaminophenol (compound of formula (2-ii-2) below) in the form of a powdery crystal.
Yield: 72.6%, purity (HPLC area percentage): 98.2%.

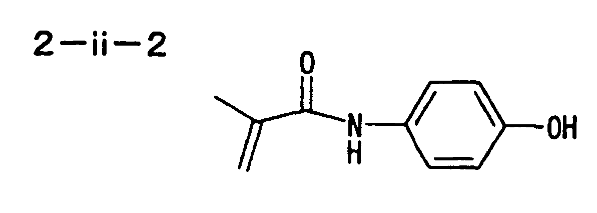

### <Production of phosphate compound 1-2>

44.9 g (0.29 mole) of phosphoryl chloride and 40.0 g of tetrahydrofuran were weighed out and mixed to produce a solution, and this solution was cooled to -5°C. To the solution, a mixed solution containing 34.6 g (0.195 mole) of compound 2-ii-2 produced in Synthesis Example 2, 29.6 g (0.29 mole) of triethylamine and 80 g of tetrahydrofuran was added dropwise over a period of 2 hours while maintaining the temperature at -5°C to 0°C. The mixture was stirred for 1.5 hours at 3°C, and disappearance of the starting materials was confirmed by liquid chromatography. Subsequently, the reaction solution was subjected to hydrolysis while adding dropwise a mixture of 125.0 g (6.9 mole) of water, 39.5 g (0.39 mole) of triethylamine and 0.3 g of tetrahydrofuran, at 3°C for 25 hours.

Then, 75.0 g of ethyl acetate, 75.0 g of toluene and 100 g of distilled water were added to the reaction solution, which was then washed and partitioned to remove an organic phase. The resulting aqueous phase was subjected twice to extraction with 300 g of n-butanol. 10 wt% aqueous hydrochloric acid was added to the resulting organic phase. After removing the aqueous phase, 200 g toluene was added to the organic phase, which was then subjected to azeotropic dehydration under reduced pressure. Thereafter, concentration of the organic phase was conducted while adding toluene in order to replace the n-butanol. The concentration was completed when a solid precipitated. The solid was filtered, washed with toluene, and dried at 40°C in a nitrogen stream to give 38.1 g of 4-methacryloylaminophenyl phosphate (compound of formula (1-2) below).
Yield: 76.0%, purity (HPLC area percentage): 95.0%.

Spectral data and results of mass analysis of the compound are as follows:
- H¹-NMR δ (DMSO-d6): 1.94 (3H, -CH₃), 5.50 and 5.80 (2H, =CH₂), 7.01-7.63 (4H, aromatic ring), 9.77 (1H, -NH-)
- IR (cm⁻¹): 3343 and 1530 (-NHCO-), 1650 (C=C), 1212 (P=O), 9.84 (P-OH)
- FD-MS: 258 (M+H), 515 (2M+H)

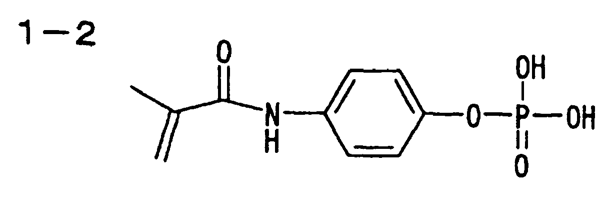

### Synthesis Example 3

### <Production of unsaturated double bond-containing compound 2-ii-3>

32.7 g (0.30 mole) of m-aminophenol and 80 g of N,N-dimethylacetamide were mixed and dissolved to produce a solution. To the solution, 29.8 g (0.285 mole) of methacrylic acid chloride was added dropwise at 50°C over a period of 2 hours. After heating the mixture at 50°C for another 2 hours, it was confirmed by liquid chromatography that the reaction had been almost completed. The solution was then cooled to room temperature. The solution was extracted and partitioned with 150 g of ethyl acetate. The resulting organic phase was left overnight, and a solid that had precipitated was filtered, washed with toluene, and dried at 40°C in a nitrogen stream to give 26.0 g (0.147 mole) of 3-methacryloylaminophenol (compound of formula (2-ii-3) below).
Yield: 51.6%, purity (HPLC area percentage): 99.3%.

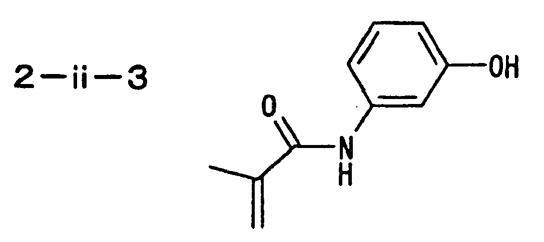

### <Production of phosphate compound 1-3>

32.2 g (0.21 mole) of phosphoryl chloride and 30.0 g of tetrahydrofuran were weighed out and mixed to produce a solution, and this solution was cooled to -5°C. To the solution, a mixed solution containing 34.6 g (0.195 mole) of compound 2-ii-3 produced in Synthesis Example 3, 21.3 g (0.21 mole) of triethylamine and 60 g of tetrahydrofuran was added dropwise over a period of 2 hours while maintaining the temperature at -5°C to 0°C. The mixture was stirred at 3°C for 1.5 hours, and disappearance of the starting materials was confirmed by liquid chromatography. Then, the reaction solution was subjected to hydrolysis while adding dropwise a mixture of 90.0 g (5.0 moles) of water, 28.3 g (0.28 mole) of triethylamine and 0.3 g of tetrahydrofuran, at 3°C for 25 hours.

Then, the reaction solution was subjected twice to extraction with 200 g of n-butanol, and 10 wt% aqueous hydrochloric acid was added to the resulting organic phase. After removing the aqueous phase, 200 g of toluene was added to the organic phase, which was then subjected to azeotropic dehydration under reduced pressure. Concentration of the organic phase was conducted while adding toluene so as to replace the n-butanol. As a result, 27.2 g of 3-methacryloylaminophenyl phosphate (compound of formula (1-3) below) was obtained in the form of a crystal.
Yield: 75.8%, purity (HPLC area percentage): 98.0%.

Spectral data and results of mass analysis of the compound are as follows:
- H¹-NMR δ (DMSO-d6): 1.96 (3H, -CH₃), 5.52 and 5.83 (2H, =CH₂), 6.90-7.66 (4H, aromatic ring), 9.90 (1H, -NH-), 11.4 (2H, P-OH)
- IR (cm⁻¹): 3384 and 1605 (-NHCO-), 1655 (C=C), 1189 (P=O), 1000-900 (P-OH)
- FD-MS: 258 (M+H), 515 (2M+H)

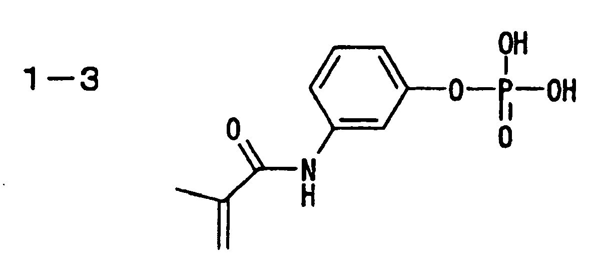

### Synthesis Example 4

### <Production of unsaturated double bond-containing compound 2-ii-4>

32.7 g (0.30 mole) of m-aminophenol and 80 g of N,N-dimethylacetamide were mixed and dissolved to produce a solution. To the solution, 25.8 g (0.285 mole) of acrylic acid chloride was added dropwise at 50°C over a period of 2 hours. After heating the mixture at 50°C for another 2 hours, it was confirmed by liquid chromatography that the reaction had been almost completed. The reaction solution was then cooled to room temperature.

The reaction solution was washed and partitioned with 200 g of ethyl acetate and 200 g of distilled water, and the organic phase was washed with NaHCO₃ water. Thereafter, 200 g of toluene was added to the organic phase, and the solvent was distilled away under reduced pressure. Concentration of the organic phase was conducted while further adding 400 g of toluene in twice. The concentration was completed when a solid precipitated. The resulting solid was subjected to sludging with 500 g of toluene, and was then filtered and washed to give a moistened material. It was dried at 40°C in a nitrogen stream to give 28.9 g (0.177 mole) of 3-acryloylaminophenol (compound of formula (2-ii-4) below) in the form of a powdery crystal.
Yield: 62.1%, purity (HPLC area percentage): 99.2%.

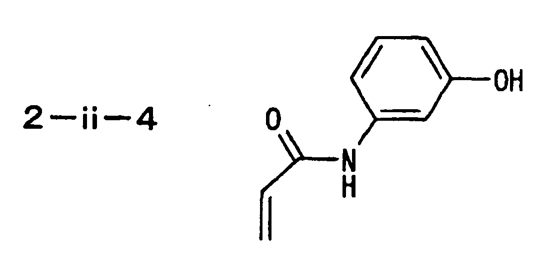

### <Production of phosphate compound 1-4>

39.1 g (0.255 mole) of phosphoryl chloride and 30.0 g of tetrahydrofuran were weighed out and mixed to produce a solution, and this solution was cooled to -5°C. To the solution, a mixed solution containing 27.7 g (0.17 mole) of compound 2-ii-4 produced in Synthesis Example 4, 25.8 g (0.255 mole) of triethylamine and 60 g of tetrahydrofuran was added dropwise over a period of 2 hours while maintaining the temperature at -5°C to 0°C. The mixture was then stirred at 3°C for 1.5 hours, and disappearance of the starting materials was confirmed by liquid chromatography. Subsequently, the reaction solution was subjected to hydrolysis while adding dropwise a mixture of 110.0 g (6.1 moles) of water, 34.4 g (0.34 mole) of triethylamine and 0.3 g of tetrahydrofuran, at 3°C for 25 hours.

Then, the reaction solution was subjected twice to extraction with 200 g of n-butanol, and 10 wt% aqueous hydrochloric acid was added to the resulting organic phase. After removing the aqueous phase, 200 g of toluene was added to the organic phase, which was then subjected to azeotropic dehydration under reduced pressure. Concentration of the organic phase was conducted while adding toluene so as to replace the n-butanol. As a result, 42.2 g of 3-acryloylaminophenyl phosphate (compound of formula (1-4) below) was obtained in the form of an aqueous viscous solution. The water content in the compound was 24 wt%.
Yield: 72.6%, purity (HPLC area percentage): 97.7%.

Spectral data and results of mass analysis of the compound are as follows:
- H¹-NMR δ (DMSO-d6): 5.70-5.73 and 6.22-6.26 (2H, =CH₂), 6.36-6.44 (1H, =CH), 6.87-7.61 (4H, aromatic ring), 10.30 (1H, -NH-)
- IR (cm⁻¹): 1600 (C=O), 1659 (C=C), 1215 (P=O), 951 (P-OH)
- FAB-MS: 242 (M-H), 485 (2M-H)

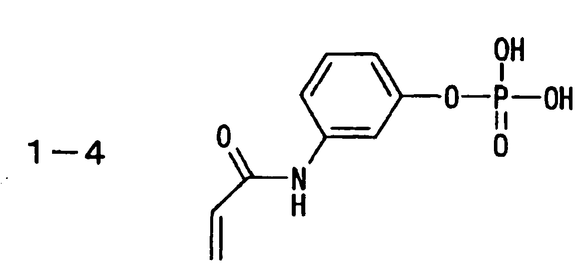

### Synthesis Example 5

### <Production of unsaturated double bond-containing compound 2-ii-5>

31.5 g (0.20 mole) of p-nitrochlorobenzene and 44.04 g (0.40 mole) of hydroquinone were dissolved in 150 g of dimethylformamide, then 13.8 g (0.10 mole) of potassium carbonate was added thereto, and the solution was heated to 120°C to cause reaction. 13 hours after, disappearance of the starting material (p-nitrochlorobenzene) was confirmed, and then the reaction solution was cooled.

The reaction solution was extracted and partitioned by adding 300 g of distilled water and 500 g of toluene. The resulting organic phase was concentrated to yield a residue, which was then dissolved in ethanol. The resultant was subjected to re-precipitation by adding distilled water, then filtered and dried to give 40.2 g of 4-(4'-nitrophenoxy)phenol (yield in partial purification: 87.0%) in the form of a brown crystal. 20.0 g of the crystal was dissolved in 120 g of ethanol and was then subjected to catalytic hydrogenation reduction in the presence of 5% Pd/C catalyst. After the completion of the reaction, the catalyst was filtered off, and the resulting solution was subjected to re-precipitation by adding distilled water, then filtering and drying to give 14.8 g of 4-(4'-aminophenoxy)phenol in the form of a crystal. The yield in partial purification was 85.0%, and the purity (HPLC area percentage) was 98.0%.

13.5 g (0.065 mole) of the resulting 4-(4'-aminophenoxy)phenol and 40.0 g of N,N-dimethylacetamide were mixed and dissolved to give a solution, and 7.81 g (0.0748 mole) of methacrylic acid chloride was added dropwise to the solution at 50°C over a period of 1.5 hours. After heating the mixture at 50°C for another 2 hours, it was confirmed by liquid chromatography that the reaction had been almost completed, and then the reaction solution was cooled to room temperature. The reaction solution was diluted with 100 g of ethyl acetate and 100 g of distilled water, and the aqueous phase was removed. The organic phase was washed with 5% sodium bicarbonate water, and the solvent was distilled away from the organic phase under reduced pressure while adding toluene. A solid that had precipitated was collected by filtration, sludge-washed with toluene, and dried to give 15.9 g (0.059 mole) of 4-(4'-methacryloylaminophenoxy) phenol (compound of formula (2-ii-5) below) in the form of a powdery crystal.
Yield: 90.8%, purity (HPLC area percentage): 97.6%.

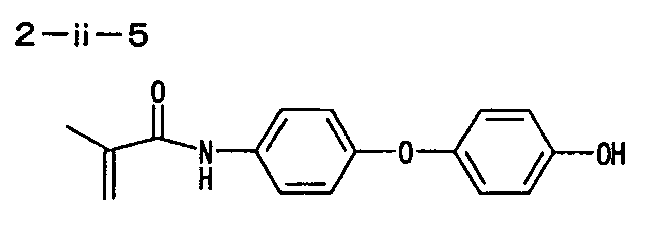

### <Production of phosphate compound 1-5>

12.7 g (0.083 mole) of phosphoryl chloride and 12 g of tetrahydrofuran were mixed to produce a solution, and this solution was cooled to -5°C. To the solution, a mixed solution containing 15.9 g (0.059 mole) of compound 2-ii-5 produced in Synthesis Example 5, 8.36 g (0.083 mole) of triethylamine and 24 g of tetrahydrofuran was added dropwise over a period of 1.5 hours while maintaining the temperature at 0°C or less, then the mixture was stirred at 0 °C to 3°C for 1 hour to complete the reaction. Then, the reaction solution was subjected to hydrolysis while adding dropwise a mixture of 41 g (2.28 mole) of water, 11.9 g (0.118 mole) of triethylamine and 0.10 g of tetrahydrofuran, at 3°C for 20 hours.

The resulting reaction solution was subjected to extraction with 200 g of n-butanol and 100 g of distilled water, and the extract was washed with water and partitioned to recover the organic phase. The solvent was distilled away from the organic phase under reduced pressure, and the residue was subjected to recrystallization with methanol/water to give 14.8 g of 4-(4"-methacryloylaminophenoxy)phenyl phosphate (compound of formula (1-5) below).
Yield: 72%, purity (HPLC area percentage): 95%.

Spectral data and results of mass analysis of the compound are as follows:
- H¹-NMR δ (DMSO-d6): 1.96 (3H, -CH₃), 5.51 and 5.82 (2H, =CH₂), 6.97-7.72 (8H, aromatic ring), 9.83 (1H, -NH-), 10.34 (P-OH)
- IR (cm⁻¹): 1500 (C=O), 1644 (C=C), 1201 (P=O), 985 (P-OH)
- FD-MS: 350 (M+H), 699 (2M+H)

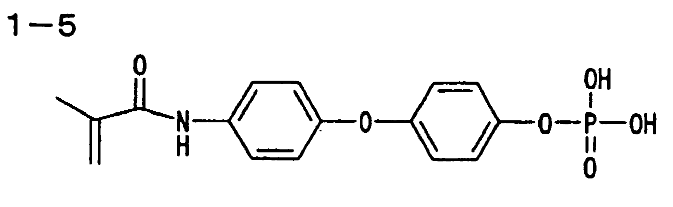

### Example 1

### <Preparation of polymerizable composition 1>

5 parts by weight of the unsaturated double bond-containing phosphate compound of formula (1-1) produced in Synthesis Example 1, 80 parts by weight of 2,4,4-trimethyl-1,6-(2-methacryloyloxyethyloxycarbonylamino)hexane (UDMA, manufactured by Shin-Nakamura Chemical Co., Ltd.), 20 parts by weight of neopentyl glycol dimethacrylate (NPG, manufactured by Shin-Nakamura Chemical Co., Ltd.), 80 parts by weight of silica fine powder (RM-50, manufactured by Nippon Aerosil Co., Ltd.), and 0.01 part by weight of methoxy hydroquinone were weighed out respectively and mixed well in an agate mortar to give a uniform paste. Polymerizable composition 1 in the form of a colorless semitransparent paste was thus obtained.

### Examples 2 to 5

### <Preparation of polymerizable compositions 2 to 5>

Polymerizable compositions 2 to 5 in the form of a colorless semitransparent paste were obtained in the same manner as in Example 1, except that the unsaturated double bond-containing phosphate compounds of formulae (1-2) to (1-5) produced in Synthesis Examples 2 to 5 were used in place of the unsaturated double bond-containing phosphate compound of formula (1-1) produced in Synthesis Example 1.

### Examples 6 to 10

### <Curing of polymerizable compositions 1 to 5 and evaluation of physical properties of cured products>

An adhesion test was conducted according to a known method described in documents (for example, JP-A No. 2-117906). Namely, the surface of a stainless steel plate (SUS304, 10 mm ×10 mm ×3 mm) was polished with emery paper #600, and then subjected to ultrasonic cleaning in acetone for 10 minutes using ultrasonic cleaning equipment. A mold formed from polytetrafluoroethylene of 2 mm in thickness with holes having a diameter of 5 mm was bonded to the surface of the stainless steel plate with a double-sided adhesive tape.

Polymerizable compositions 1 to 5 prepared in Examples 1 to 5 were filled in the mold, respectively, and then polymerized and cured in an inert oven in a nitrogen atmosphere by heating at 50°C for 3 hours, at 60°C for 3 hours, at 70°C for 1 hour, at 80°C for 1 hour, and at 90°C for 3 hours in this order.

After the curing had been completed, the mold was removed from the samples and the adhesion between the stainless steel plate and the resin cured product was evaluated. As a result, the adhesion was favorable and the physical properties had no problem in practical use.

### Industrial Applicability

By using the phosphate compound of the invention and/or a metal salt of the phosphate compound, it is possible to provide a dental material and a dental composition, such as a bonding material and a dental adhesives/dental luting agent that exhibits excellent storage stability and handleability, and higher adhesiveness and bond durability.

The dental material of the present invention encompasses general dental materials formed from an organic material used in dental therapy, for example, crown materials such as crown resin and prosthetic tooth, dental filling materials such as composite resin, root canal filling material and bonding material, and dental adhesives/dental luting agents such as resin cement and orthodontic adhesive, as well as Fischer sealant, coating material, crown/bridge/inlay resin, dental abutment construction material, denture base resin, and denture base repairing resin.

Further, the phosphate compound of the invention and/or a metal salt of the phosphate compound is useful as a modifier for meth(acrylic) resin, and is also usable in various kinds of coating materials, adhesive materials and molding materials, other than the dental materials.

## Claims

1. A phosphate compound selected from the group consisting of: 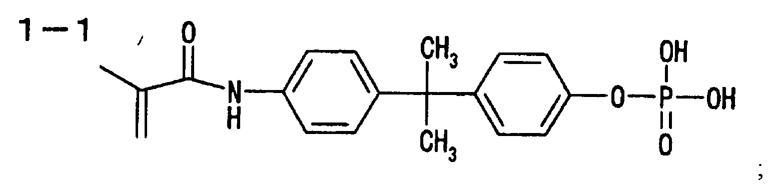 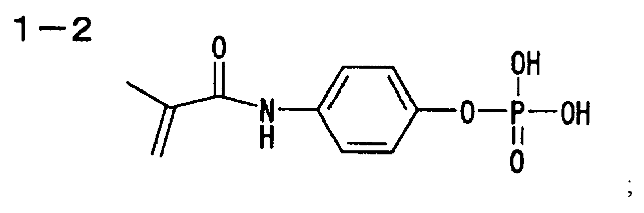 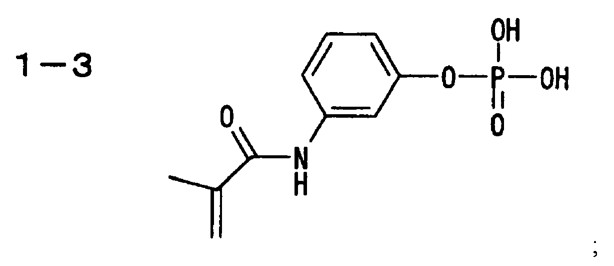 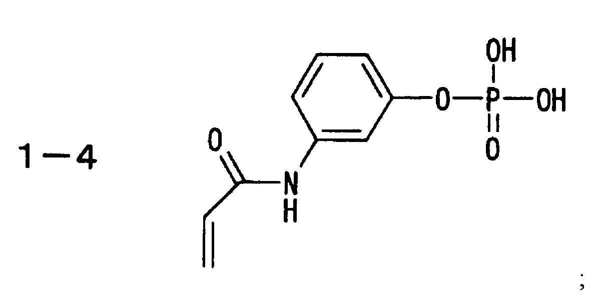 and 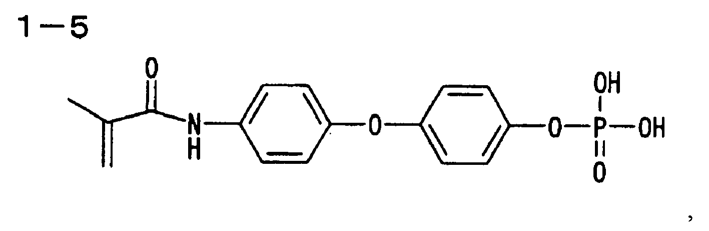 or a metal salt thereof.

2. The phosphate compound or the metal salt of claim 1, wherein the metal salt comprises a Li salt, a Na salt, a K salt, a Cu salt, an Ag salt, a Mg salt, a Ca salt, a Sr salt, a Zn salt, a Ba salt, an Al salt, a Ti salt, a Zr salt, a Sn salt, a Fe salt, a Ni salt or a Co salt.

3. A dental material comprising a phosphate compound according to claim 1 or claim 2, or a metal salt of the phosphate compound.

4. The dental material of claim 3, wherein the metal salt comprises a Li salt, a Na salt, a K salt, a Mg salt, a Ca salt or a Ba salt.

5. A dental composition comprising a polymerizable compound and a polymerization initiator, the polymerizable compound comprising a phosphate compound according to claim 1 or claim 2, or a metal salt of the phosphate compound.

6. The dental composition of claim 5, wherein the metal salt comprises a Li salt, a Na salt, a K salt, a Mg salt, a Ca salt or a Ba salt.

7. The dental composition of claim 5 or 6, further comprising a (meth)acrylate compound or a (meth)acrylic acid amide compound as a polymerizable compound.

8. The dental composition of any of claims 5 to 7, further comprising an acid group-containing monomer as a polymerizable compound.

9. The dental composition of any of claims 5 to 8, further comprising a filler.

10. The dental composition of any of claims 5 to 9, wherein the polymerization initiator comprises a heat polymerization initiator, an autopolymerization initiator or a photopolymerization initiator.

11. A modifier for a (meth)acrylic resin, the modifier comprising a phosphate compound according to claim 1 or claim 2, or a metal salt of the phosphate compound.

12. A coating material comprising a phosphate compound according to claim 1 or claim 2, or a metal salt of the phosphate compound.

13. An adhesive material comprising a phosphate compound according to claim 1 or claim 2, or a metal salt of the phosphate compound.

14. A molding material comprising a phosphate compound according to claim 1 or claim 2, or a metal salt of the phosphate compound.

## Patentansprüche

1. Phosphatverbindung, aus folgender Gruppe ausgewählt: 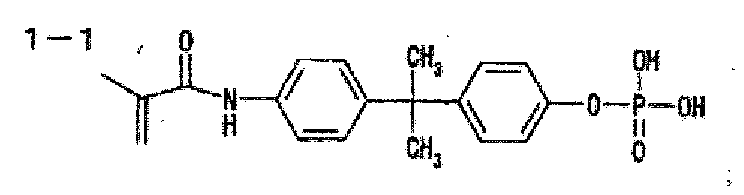 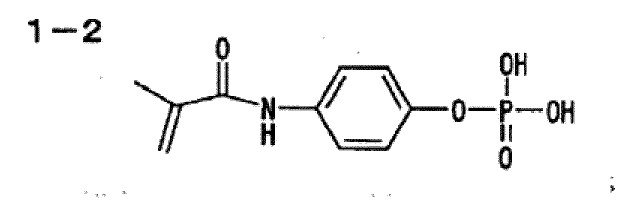 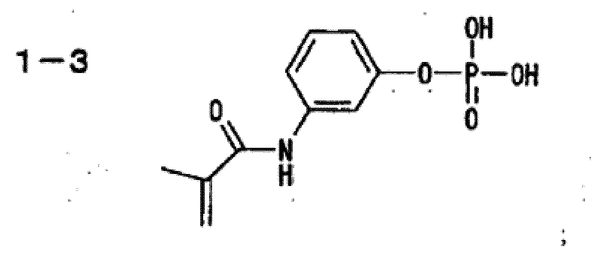 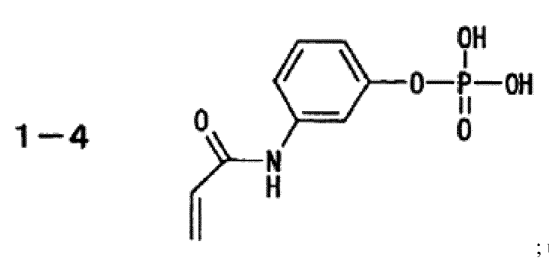 und 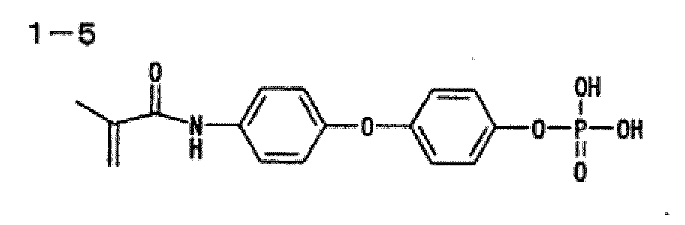 oder ein Metallsalz davon.

2. Phosphatverbindung oder Metallsalz nach Anspruch 1, wobei das Metallsalz ein Li-Salz, ein Na-Salz, ein K-Salz, ein Cu-Salz, ein Ag-Salz, ein Mg-Salz, ein Ca-Salz, ein Sr-Salz, ein Zn-Salz, ein Ba-Salz, ein Al-Salz, ein Ti-Salz, ein Zr-Salz, ein Sn-Salz, ein Fe-Salz, ein Ni-Salz oder ein Co-Salz umfasst.

3. Dentalmaterial, das eine Phosphatverbindung nach Anspruch 1 oder Anspruch 2 oder ein Metallsalz der Phosphatverbindung umfasst.

4. Dentalmaterial nach Anspruch 3, wobei das Metallsalz ein Li-Salz, ein Na-Salz, ein K-Salz, ein Mg-Salz, ein Ca-Salz oder ein Ba-Salz umfasst.

5. Dentalzusammensetzung, die eine polymerisierbare Verbindung und einen Polymerisationsinitiator umfasst, wobei die polymerisierbare Verbindung eine Phosphatverbindung nach Anspruch 1 oder Anspruch 2 oder ein Metallsalz der Phosphatverbindung umfasst.

6. Dentalzusammensetzung nach Anspruch 5, wobei das Metallsalz ein Li-Salz, ein Na-Salz, ein K-Salz, ein Mg-Salz, ein Ca-Salz oder ein Ba-Salz umfasst.

7. Dentalzusammensetzung nach Anspruch 5 oder 6, die weiters eine (Meth)acrylatverbindung oder eine (Meth)acrylsäureamidverbindung als polymerisierbare Verbindung umfasst.

8. Dentalzusammensetzung nach einem der Ansprüche 5 bis 7, die weiters ein Säuregruppen-hältiges Monomer als polymerisierbare Verbindung umfasst.

9. Dentalzusammensetzung nach einem der Ansprüche 5 bis 8, die weiters einen Füllstoff umfasst.

10. Dentalzusammensetzung nach einem der Ansprüche 5 bis 9, wobei der Polymerisationsinitiator einen Wärmepolymerisationsinitiator, einen Autopolymerisationsinitiator oder einen Photopolymerisationsinitiator umfasst.

11. Modifikator für ein (Meth)acrylharz, wobei der Modifikator eine Phosphatverbindung nach Anspruch 1 oder Anspruch 2 oder ein Metallsalz der Phosphatverbindung umfasst.

12. Beschichtungsmaterial, das eine Phosphatverbindung nach Anspruch 1 oder Anspruch 2 oder ein Metallsalz der Phosphatverbindung umfasst.

13. Klebstoffmaterial, das eine Phosphatverbindung nach Anspruch 1 oder Anspruch 2 oder ein Metallsalz der Phosphatverbindung umfasst.

14. Formmasse, die eine Phosphatverbindung nach Anspruch 1 oder Anspruch 2 oder ein Metallsalz der Phosphatverbindung umfasst.

## Revendications

1. Composé phosphate choisi dans le groupe constitué par 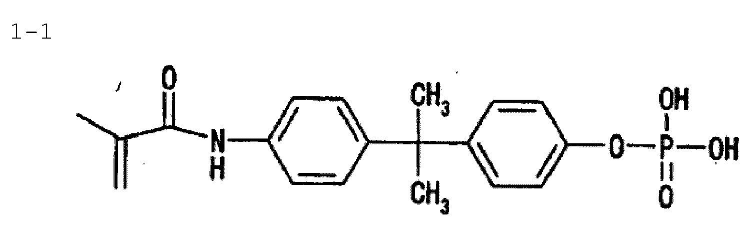 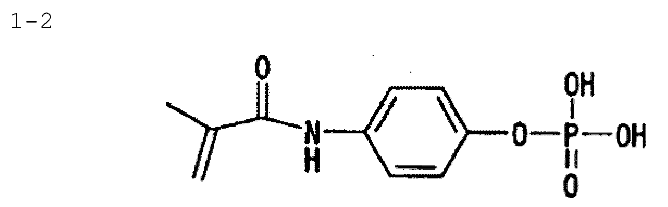 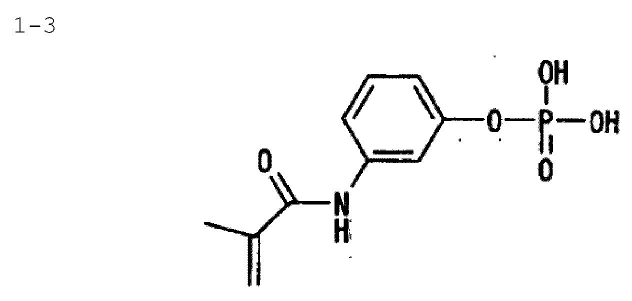 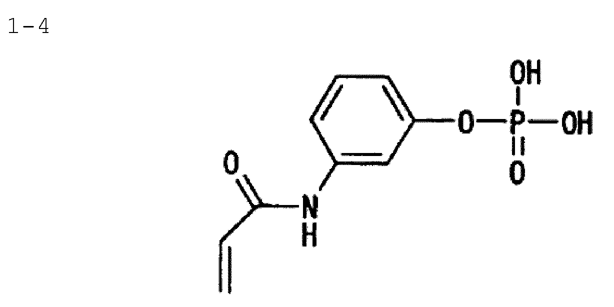 et 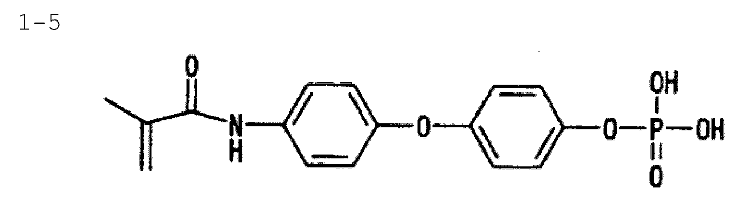 ou un sel métallique de celui-ci.

2. Composé phosphate ou sel métallique selon la revendication 1, dans lequel le sel métallique comprend un sel de Li, un sel de Na, un sel de K, un sel de Cu, un sel d'Ag, un sel de Mg, un sel de Ca, un sel de Sr, un sel de Zn, un sel de Ba, un sel d' Al, un sel de Ti, un sel de Zr, un sel de Sn, un sel de Fe, un sel de Ni ou un sel de Co.

3. Matériau dentaire comprenant un composé phosphate selon la revendication 1 ou la revendication 2, ou un sel métallique du composé phosphate.

4. Matériau dentaire selon la revendication 3, dans lequel le sel métallique comprend un sel de Li, un sel de Na, un sel de K, un sel de Mg, un sel de Ca ou un sel de Ba.

5. Composition dentaire comprenant un composé polymérisable et un amorceur de polymérisation, le composé polymérisable comprenant un composé phosphate selon la revendication 1 ou la revendication 2, ou un sel métallique du composé phosphate.

6. Composition dentaire selon la revendication 5, dans laquelle le sel métallique comprend un sel de Li, un sel de Na, un sel de K, un sel de Mg, un sel de Ca ou un sel de Ba.

7. Composition dentaire selon la revendication 5 ou 6, comprenant en outre un composé (méth)acrylate ou un composé amide d'acide (méth)acrylique en tant que composé polymérisable.

8. Composition dentaire selon l'une quelconque des revendications 5 à 7, comprenant en outre un monomère contenant un groupe acide en tant que composé polymérisable.

9. Composition dentaire selon l'une quelconque des revendications 5 à 8, comprenant en outre une charge.

10. Composition dentaire selon l'une quelconque des revendications 5 à 9, dans lequel l'amorceur de polymérisation comprend un amorceur de polymérisation à la chaleur, un amorceur d'auto-polymérisation ou un amorceur de photopolymérisation.

11. Modificateur pour une résine (méth)acrylique, le modificateur comprenant un composé phosphate selon la revendication 1 ou la revendication 2, ou un sel métallique du composé phosphate.

12. Matériau de revêtement comprenant un composé phosphate selon la revendication 1 ou la revendication 2, ou un sel métallique du composé phosphate.

13. Matériau adhésif comprenant un composé phosphate selon la revendication 1 ou la revendication 2, ou un sel métallique du composé phosphate.

14. Matériau de moulage comprenant un composé phosphate selon la revendication 1 ou la revendication 2, ou un sel métallique du composé phosphate.
